(19) Europäisches Patentamt
European Patent Office
Office européen des brevets

(11) Veröffentlichungsnummer: **0 120 208**
**B1**

(12) # EUROPÄISCHE PATENTSCHRIFT

(45) Veröffentlichungstag der Patentschrift: **22.03.89**

(21) Anmeldenummer: **84100707.3**

(22) Anmeldetag: **24.01.84**

(51) Int. Cl.⁴: **C 12 N 9/06**, C 12 P 13/22, C 12 P 13/12 // (C12N9/06, C12R1:13)

(54) **Mikrobiologisch hergestellte L-Phenylalanin-Dehydrogenase, Verfahren zu ihrer Gewinnung und ihre Verwendung.**

(30) Priorität: **01.03.83 DE 3307095**

(43) Veröffentlichungstag der Anmeldung: **03.10.84 Patentblatt 84/40**

(45) Bekanntmachung des Hinweises auf die Patenterteilung: **22.03.89 Patentblatt 89/12**

(84) Benannte Vertragsstaaten: **BE CH DE FR GB LI NL SE**

(56) Entgegenhaltungen:
**EP-A- 0 023 346**
**DE-A- 2 041 418**
**US-A- 3 036 958**

**CHEMICAL ABSTRACTS, Band 57, 23. Juli 1962, Spalten 2679h - 2280b, Columbus, Ohio, US; KO AIDA et al.: "Amino acid fermentation. VI. Preparation of L-tryptophan by bacterial reductive amination", & HAKKO TO TAISHA 1959, 1, 94-8**
**INDUSTRIAL MICROBIOLOGY ABSTRACTS, Band 1, Nr. 2, 1965, Seite 5, Nr. 138; I. CHIBUTA et al.: "Production of L-phenylalanine from D-phenylalanine", & APPL.. MICROBIOL. 1965, 13, 618**
**CHEMICAL ABSTRACTS, Band 75, Nr. 13, 27. September 1971, Seite 115, Nr. 85543a, Columbus, Ohio, US; K.**

(73) Patentinhaber: **Degussa Aktiengesellschaft, Weissfrauenstrasse 9, D-6000 Frankfurt am Main 1 (DE)**
Patentinhaber: **Gesellschaft für biotechnologische Forschung mbH (GBF), Mascheroder Weg 1, D-3300 Braunschweig-Stöckheim (DE)**

(72) Erfinder: **Leuchtenberger, Wolfgang, Dr., Dipl.-Chem., Geschwister-Scholl-Strasse 1, D-6454 Bruchköbel (DE)**
Erfinder: **Kula, Maria-Regina, Dr., Forstweg 15, D-3340 Wolfenbüttel (DE)**
Erfinder: **Hummel, Werner, Dr., Glückstrasse 3, D-3300 Braunschweig (DE)**
Erfinder: **Schütte, Horst, Nordring 29 A, D-3320 Salzgitter 51 (DE)**

(56) Entgegenhaltungen: (Fortsetzung)
**PORALLA: "Induction of a dehydrogenase activity for branched chain amino acids in Bacillus subtilis", & ARCH. MIKROBIOL. 1971, 77(4), 339-43**
**CHEMICAL ABSTRACTS, Band 80, Nr. 19, 13. Mai 1974, Seite 287, Nr.106865c, Columbus, Ohio, US; & JP - A - 73 91 263 (SANRAKU OCEAN CO. LTD.) 28.11.1973**
**CHEMICAL ABSTRACTS, Band 97, Nr. 25, 20. Dezember 1982, Seite 349, Nr. 210708r, Columbus, Ohio, US; G. SAURET-IGNAZI: "Generation of hydrogen peroxide during the oxidation of L-phenylalanine by Proteus mirabilis isolated membranes", & BIOCHIMIE 1982, 64(10), 891-7**

EP 0 120 208 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein bisher nicht beschriebenes Enzym, das die folgende Umsetzung katalysiert:

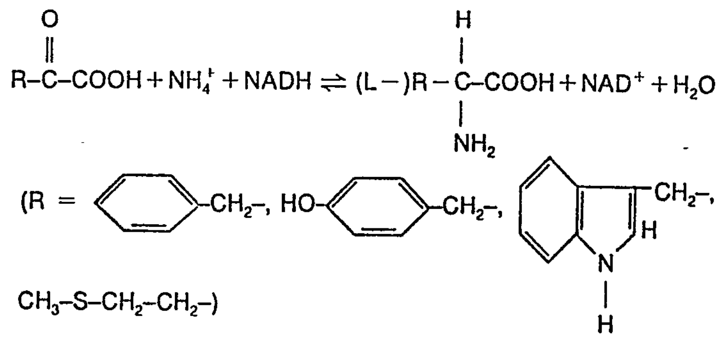

ein Verfahren zu seiner Gewinnung und seine Verwendung.

Es sind Enzyme bekannt, mittels derer L-Glutaminsäure und verschiedene andere aliphatische L-$\alpha$-Aminocarbonsäuren, beispielsweise L-Alanin, L-Valin, L-Leucin und L-Isoleucin, durch reduktive Aminierung entsprechender $\alpha$-Ketocarbonsäuren in Gegenwart von Ammoniumionen und eines Coenzyms hergestellt werden können. Während L-Glutaminsäure-Dehydrogenase eine zentrale Rolle im Stickstoff-Stoffwechsel spielt und ubiquitär vorkommt, ist die reduktive Aminierung anderer aliphatischer $\alpha$-Ketocarbonsäuren vor allem für Enzyme aus der Gattung Bacillus beschrieben. Keine der bisher bekannten L-Aminosäure-Dehydrogenasen katalysiert jedoch die Umwandlung von aromatischen oder heteroaromatischen $\alpha$-Ketocarbonsäuren in die entsprechenden L-$\alpha$-Aminocarbonsäuren.

Die erfindungsgemäße L-Phenylalanin-Dehydrogenase ist gekennzeichnet durch die folgenden Eigenschaften:

a) katalysiert die reduktive Aminierung von Phenylbrenztraubensäure zu L-Phenylalanin in Gegenwart von Ammoniumionen und mit NADH (Nicotinamid-adenin-dinucleotid) als Coenzym,

b) katalysiert die reduktive Aminierung weiterer $\alpha$-Ketocarbonsäuren zu den entsprechenden $\alpha$-Aminocarbonsäuren, insbesondere von $\beta$-Hydroxyphenylbrenztraubensäure zu L-Tyrosin, von Indolylbrenztraubensäure zu L-Tryptophan und von 2-Keto-4-(methylmercapto)-buttersäure zu L-Methionin, in Gegenwart von Ammoniumionen und mit NADH als Coenzym,

c) katalysiert die oxidative Desaminierung von L-Phenylalanin, L-Tyrosin, L-Tryptophan und L-Methionin mit $NAD^+$ als Coenzym,

d) einen optimale pH-Bereich für die reduktive Aminierung von $8{,}5 \pm 1$ und

e) einen optimalen pH-Bereich für die oxidative Desaminierung von $10 \pm 1$

und erhältlich durch Züchtung des Stammes Brevibacterium DSM 2448 und Gewinnung daraus.

Die erfindungsgemäße L-Phenylalanin-Dehydrogenase kann mittels eines Brevibacterium-Stammes gewonnen werden, der am 19. 08. 1982 bei der Deutschen Sammlung von Mikroorganismen in Göttingen unter der Nummer 2448 hinterlegt wurde. Der Stamm zeichnet sich durch die folgenden morphologischen und biologischen Eigenschaften aus:

Brevibacterium species DSM 2448 wächst in kurzen, grampositiven Stäbchen, die mit zunehmendem Alter in kokkoide Formen übergehen. Die Zellen sind unbeweglich und bilden keine Sporen. Wachstum erfolgt strikt aerob. Aus Glucose wird keine Säure gebildet. Katalase- und Nitratreduktion sind positiv, Harnstoffspaltung positiv, Gelatine-, Casein- und Stärkeabbau negativ, $H_2S$-Bildung negativ, Wachstum beu 41 °C negativ. Als Zellwandzucker wurden Arabinose, Mannose und Galactose nachgewiesen. Der Organismus ist strikt aerob und unterscheidet sich so von Corynebacterien. Die Zellwand enthält meso-Diaminopimelinsäure und unterscheidet sich so von der Gattung Arthrobacter.

Das Spektrum der Cytochrome unterscheidet den Organismus weiterhin von der Gattung Microbacterien. Der neugefundene Organismus Brevibacterium species DSM 2448 kann zur Zeit keiner bekannten Art innerhalb der Gattung Brevibacterium zugeordnet werden. Eine Reihe von Stämmen verschiedener Arten von Brevibacterium und verwandter Gattungen, die zum Teil als Aminosäureproduzenten bekannt sind, wurde zusätzlich unter Bedingungen, unter denen Brevibacterium species DSM 2448 L-Phenylalanin-Dehydrogenase bildet, angezogen und auf die Fähigkeit zur reduktiven Aminierung von Phenylbrenztraubensäure in Gegenwart vom Ammoniumionen und NADH geprüft. In keinem Falle konnte die L-Phenylalanin-Dehydrogenase nachgewiesen werden.

Zur Gewinnung der erfindungsgemäßen L-Phenylalanin-Dehydrogenase wird Brevibacterium species DSM 2448 in einem wäßrigen Nährmedium, das eine Quelle für Kohlenstoff und Stickstoff, Thiamin, Mineralsalze, sowie einen Induktor enthält, bei einem pH zwischen 6,5 und 7,5 und einer Temperatur zwischen 25 und 32 °C aerob kultiviert, die Zellmasse abgetrennt und das Enzym aus den Zellen isoliert. Als Induktoren können beispielsweise L-Phenylalanin, D-Phenylalanin, D,L-Phenylalanin, D,L-Phenylalaninester oder L-Histidin eingesetzt werden.

Die erfindungsgemäße L-Phenylalanin-Dehydrogenase läßt sich durch einen Aufschluß der Zellen nach herkömmlichen Methoden, z. B. Ultraschallbehandlung oder Naßvermahlung, und Abtrennen der unlöslichen Zellfragmente in löslicher Form gewinnen. Die Enzymaktivität wird mit einem photometrischen Test bestimmt, indem die Abnahme der Extinktion von NADH bei 340 nm gewessen wird. Der Testansatz enthält 0,7 M Ammoniumchlorid, mit Ammoniak auf pH 8,5 eingestellt, 0,20 mM NADH, Phenylpyruvat und limitierende Mengen von Enzym. Die Enzymaktivität wird in internationalen Einheiten (U) angegeben, wobei eine Einheit die Abnahme von 1 μMol NADH pro Minute bedeutet. Für die Berechnung wird ein molarer Extinktionscoefficient von 6,22 × $10^3$ bei 340 nm eingesetzt. Bereits mit Rohextrakt als Katalysator werden pro Mol verbrauchtes NADH 0,92 Mol L-Phenylalanin am Aminosäureanalysator unter den Testbedingungen nachgewiesen. Daß es sich dabei nicht um eine gekoppelte Reaktion einer Glutamat-Dehydrogenase mit nachfolgender Transaminierung von Phenylpyruvat handelt, zeigt die Umsetzung im Membranreaktor, wo über 110 Stunden eine kontinuierliche Bildung von L-Phenylalamin aus Phenylpyruvat unter Regenerierung des eingesetzten molekulargewichtsvergrößerten NADH mit Formiat-Dehydrogenase nachgewiesen werden konnte. Die Reaktionsgeschwindigkeit der katalysierten Reaktion wurde in Abhängigkeit vom pH gemessen. Die reduktive Aminierung hat ein Maximum bei pH 8,5, die oxidative Desaminierung steigt bis etwa pH 10,5 an. Die höchste Reaktionsgeschwindigkeit wird bei einer Konzentration an NADH von etwa 0,3 mMol/l gefunden. Die Reaktionsgeschwindigkeit der Umsetzung steigt mit zunehmender Ammoniumionen-Konzentration bis mindestens 0,5 M Ammoniumionen noch an. Wenn die Substratkonzentration der aromatischen α-Ketocarbonsäuren bzw. der aromatischen L-Aminocarbonsäuren klein ist gegenüber ihrem $K_M$-Wert, hängt dagegen die Reaktionsgeschwindigkeit linear von der Substratkonzentration ab. Diese Tatsache kann zu einem einfachen kinetischen Test zur Bestimmung von L-Phenylalanin, L-Tyrosin, L-Tryptophan, Phenylpyruvat und Hydroxyphenylpyruvat ausgebaut werden. D-Phenylalanin wird von der L-Phenylalanin-Dehydrogenase nicht umgesetzt und ist andererseits ein Inhibitor der oxidativen Desaminierung von L-Phenylalanin. Das Im Enzymmembranreaktor durch reduktive Aminierung von Phenylpyruvat erzeugte L-Phenylalanin wurde durch Polarimetrie und Umsetzung von D-AOD (D-Aminosäure-Oxidase, Boehringer Mannheim) auf die optische Reinheit untersucht. Mit beiden Methoden konnte keine Verunreinigung durch D-Phenylalanin im Produkt nachgewiesen werden. Das Produkt bestand mindestens zu 99,99% aus dem L-Isomeren.

Wenn Brevibacterium species DSM 2448 auf Hefeextrakt angezogen wird, katalysiert der Rohextrakt die Umsetzung einer Reihe aromatischer bzw. heteroaromatischer, aber auch aliphatischer α-Ketocarbonsäuren zu den entsprechenden α-Aminocarbonsäuren. Dieses breite Spektrum am umsetzbaren Substraten läßt vermuten, daß unter diesen Wachstumsbedingungen mehrere Enzymaktivitäten im Rohextrakt vorhanden sind.

Durch fraktionierte Fällung mit Ammoniumsulfat und nachfolgende Chromatographie unter Aussalzungsbedingungen mit einem absteigenden Ammoniumsulfatgradienten ergibt sich eine 52-fache Anreicherung der L-Phenylalanin-Dehydrogenase. Durch diese Chromatographie wird eine Dehydrogenase abgetrennt, die analog der L-Leucin-Dehydrogenase aus Bacillus sphaericus die Umsetzung der entsprechenden aliphatischen α-Ketocarbonsäuren zu L-Leucin, L-Norleucin, L-Valin, L-Norvalin und L-Isoleucin katalysiert. Die chromatographisch gereinigte L-Phenylalanin-Dehydrogenase katalysiert weiterhin die reduktive Aminierung der entsprecheden aromatischen α-Ketocarbonsäuren zu L-Phenylalanin, L-Tyrosin und L-Tryptophan, sowie von 2-Keto-4-(methylmercapto)-buttersäure zu L-Methionin. Bei weiteren Untersuchungen zeigt sich, daß die reduktive Aminierung von Phenylbrenztraubensäure, p-Hydroxyphenylbrenztraubensäure, Indolylbrenztraubensäure und 2-Keto-4-(methylmercapto)-buttersäure durch das gleiche Enzymprotein katalysiert wird. Dazu wird einerseits versucht, durch Züchtung in Gegenwart verschiedener α-Aminocarbonsäuren unterschiedliche Dehydrogenaseaktivitäten zu induzieren. Zu diesem Zweck wird Brevibacterium species DSM 2448 in einem Medium mit 1% Malzextrakt, 0,5% Glukose, 2 μg Thiamin/l, 0,2% $KH_2PO_4$ und verschiedenen α-Aminocarbonsäuren in einer Konzentration von 1% angezogen. Der pH-Wert des Mediums wird auf 7,4 eingestellt. Die Zellen werden nach 24 Stunden Wachstum bei 30 °C geerntet, mit Ultraschall aufgeschlossen, und in jedem Extrakt wird durch optischen Test die reduktive Aminierung von 6α-Ketocarbonsäuren getestet. Die Bildung von L-Tryptophan aus Indolylbrenztraubensäure wird wegen der Störung des optischen Testes mit diesem Substrat am Aminosäureanalysator überprüft. Die Ergebnisse sind in Tabelle 1 zusammengefaßt:

Tabelle 1

| Versuch Nr. | Induktor | $OD_{578}$ | Feuchtmasse g/l | Protein mg/ml | α-Ketocarbonsäuren* (Umsatz U/ml) 1 | 2 | 3 | 4 | 5 | 6 |
|---|---|---|---|---|---|---|---|---|---|---|
| 1 | L-Phe | 14,5 | 9,5 | 1,9 | 1,94 | 1,71 | 0,85 | 0,40 | 0 | 0 |
| 2 | D-Phe | 7,3 | 8,7 | 1,7 | 1,74 | 1,50 | 0,80 | 0,30 | 0 | 0 |
| 3 | D,L-Phe | 9,5 | 8,7 | 2,4 | 1,98 | 1,68 | 0,85 | 0,35 | 0 | 0 |
| 4 | L-His | 8,5 | 9,4 | 2,4 | 1,02 | 0,71 | 0,37 | 0,16 | 0 | 0 |
| 5 | L-Val | 18,1 | 23,3 | 3,3 | 0 | 0 | 0 | 0 | 0,15 | 1,28 |
| 6 | L-Norleu | 11,3 | 14,1 | 1,6 | 0 | 0 | 0 | 0 | 0,20 | 1,11 |
| 7 | L-Ile | 14,6 | 18,8 | 2,6 | 0 | 0 | 0 | 0 | 0,37 | 0,47 |
| 8 | L-Leu | 13,5 | 18,6 | 1,5 | 0 | 0 | 0 | 0 | 0,52 | 0,68 |
| 9 | L-Ala | 10,3 | 18,2 | 1,7 | 0 | 0 | 0 | 0 | 0 | 0 |
| 10 | L-Glu | 9,8 | 13,9 | 1,7 | 0 | 0 | 0 | 0 | 0 | 0 |
| 11 | $NH_4Cl$ | 12,1 | 11,2 | 1,1 | 0 | 0 | 0 | 0 | 0 | 0 |
| 12 | L-Phe-methylester** | 18,0 | 10,0 | 1,7 | 1,51 | nb | nb | nb | nb | nb |

* Für die reduktive Aminierung eingesetzte α-Ketocarbonsäuren:
1 Phenylbrenztraubensäure
2 p-Hydroxyphenylbrenztraubensäure
3 2-Keto-4-(methylmercapto)-buttersäure
4 Indolylbrenztraubensäure
5 2-Ketohexansäure
6 2-Keto-4-methylpentansäure
** Konzentration im Medium nur 0,5%
nb = nicht bestimmt

Dort zeigt sich, daß Brevibacterium species DSM 2448 zwei induzierbare L-Aminosäure-Dehydrogenasen produziert, eine L-Leucin-Dehydrogenase und eine L-Phenylalanin-Dehydrogenase. Die L-Leucin-Dehydrogenase setzt von den getesteten α-Ketocarbonsäuren 2-Ketohexansäure und 2-Keto-4-methylpentansäure um und wird durch L-Norleucin, L-Valin, L-Isoleucin und L-Leucin induziert. Die L-Phenylalanin-Dehydrogenase setzt Phenylbrenztraubensäure, p-Hydroxyphenylbrenztraubensäure, Indolylbrenztraubensäure und 2-Keto-4-(methylmercapto)-buttersäure um und wird durch L-Phenylalanin, D-Phenylalanin, D,L-Phenylalanin, L-Histidin und L-Phenylalanin-methylester induziert. Diese Ergebnisse stehen in Einklang mit dem Versuch zur chromatographischen Trennung der beiden L-Aminosäure-Dehydrogenasen.

Weiterhin wurde versucht, durch eine Hitzedenaturierung bei 52°C eine differentielle Desaktivierung der verschiedenen im Rohextrakt vorliegenden Dehydrogenaseaktivitäten zu erreichen. Dabei zeigte sich, daß unter diesen Bedingungen das die reduktive Aminierung von 2-Ketohexansäure und 2-Keto-4-methylpentansäure katalysierende Enzym mit einem einheitlichen kinetischen Verlauf rasch desaktiviert wird. Andererseits zeigt das die reduktive Aminierung von Phenylbrenztraubensäure, p-Hydroxyphenylbrenztraubensäure, Indolylbrenztraubensäure und 2-Keto-4-(methylmercapto)-buttersäure katalysierende Enzym eine größere Wärmestabilität mit einer Restaktivität von mehr als 50% nach 90-minütigem Erhitzen auf 52°C. Auch hier wird ein identischer Verlauf der Desaktivierungskinetik für alle geprüften Substrate beobachtet, was den Schluß zuläßt, daß diese Umsetzungen durch das gleiche Protein katalysiert werden.

Das Molekulargewicht der L-Phenylalanin-Dehydrogenase wurde durch Gelfiltration an Sephacryl-S 300 superfine nach der Methode von Andrews (Meth. Biochemical Analysis, Vol. 18, 1 bis 53 (1970)) ermittelt und zu 130 000 ± 10 000 Dalton bestimmt. Als Laufmittel diente 50 mM Kaliumphosphatpuffer, pH 7,5, 100 mM Natriumchlorid. Zur Stabilisierung der L-Phenylalanin-Dehydrogenase wurde dem Laufmittel so viel Ammoniumsulfat zugesetzt, daß eine 5% gesättigte Lösung vorlag. Die eingesetzten Eichproteine (Katalase, Rinderserumalbumin, Ovalbumin, und Chymotrypsinogen) wurden unter identischen Bedingungen chromatographiert. Unter der Bedingung der Gelfiltration wurde eine Dissoziation der L-Phenylalanin-Dehydrogenase in Untereinheiten von 66 000 ± 5 000 Dalton beobachtet.

Die erfindungsgemäße L-Phenylalanin-Dehydrogenase kann einerseits dazu verwendet werden, in an sich bekannter Weise in Gegenwart von Ammoniumionen und mit NADH als Coenzym Phenylbrenztraubensäure, p-Hydroxyphenylbrenztraubensäure, Indolylbrenztraubensäure oder 2-Keto-4-(methylmercapto)-buttersäure in die entsprechenden L-α-Aminocarbonsäuren umzuwandeln. Andererseits kann das neue Enzym aber auch dazu dienen, auf enzymatischem Wege in wäßrigen Lösungen die Konzentration von Phenylbrenztraubensäure oder p-Hydroxyphenylbrenztraubensäure, bzw. von L-Phenylalanin oder L-Tyrosin zu bestimmen. Die Bestimmung ist sehr einfach durchführbar: Es wird die Änderung der

Extinktion von NADH bei 340 nm verfolgt und dann die gesuchte Konzentration aus einer entsprechenden Eichkurve abgelesen.

Die Erfindung soll durch die nachfolgenden Beispiele näher erläutert werden. Prozentangaben bedeuten, sofern nicht anders angegeben, Gewichtsprozente.

Beispiel 1
Screening auf L-Phenylalanin-Dehydrogenase-Produzenten

14 Bodenproben von verschiedenen Standorten im Landkreis Braunschweig wurden mit steriler Salzlösung (0,9% NaCl) aufgeschlemmt, Aliquots des wäßrigen Überstandes auf Petrischalen mit festem Nährboden ausgestrichen und die Petrischalen bei 27 °C für 2 bis 3 Tage inkubiert. Der Nährboden wurde folgendermassen zubereitet: 10 g L-Phenylalanin, 4,8 g $K_2HPO_4 \cdot 3\ H_2O$, 1,5 g $KH_2PO_4$, 0,2 g $MgSO_4 \cdot 7\ H_2O$, 2 mg $CaCl_2 \cdot 2\ H_2O$, 0,4 mg $ZnSO_4 \cdot 7\ H_2O$, 0,2 mg $FeCl_3 \cdot 6\ H_2O$ und 20 g Agar wurden mit entionisiertem Wasser zu einem Liter gelöst, der pH-Wert auf 7,2 eingestellt und sterilisiert. Nach dem Abkühlen wurde 1 ml sterile Vitaminlösung nach Schlegel zugesetzt und der Nährboden in sterile Petrischalen gegossen. Organismen, die gutes Wachstum zeigten, wurden über mehrere Verdünnungsausstriche gereinigt. Unter dem Mikroskop einheitlich erscheinende Stämme wurden sodann in 100 ml Flüssigmedium in 500 ml-Erlenmeyer-Kolben mit zwei Schikanen bei 27 °C auf der Rundschüttelmaschine bei 100 Upm angezogen. Das Flüssigmedium hatte die oben angegebene Komposition, enthielt jedoch keinen Agar. Nach 2 bis 3 Tagen wurde der Inhalt des Schüttelkolbens zentrifugiert und das Sediment mit 0,05 M Kaliumphosphatpuffer (pH 7,4) gewaschen. Zellüberstand und Sediment wurde dazu mit Ultraschall 5 Minuten lang pulsierend aufgeschlossen. Unlösliche Zellbestandteile wurden durch Zentrifugation abgetrennt und der Überstand als Enzymquelle getestet. Aus den 14 Bodenproben wurden 57 Stämme isoliert, die auf L-Phenylalanin als einziger Kohlenstoff- und Stickstoffquelle wuchsen. Hiervon zeigte ein Stamm (Brevibacterium species DSM 2448) die gewünschte Enzymaktivität. Zum Nachweis des Enzyms wurde ein photometrischer Test benutzt. Als Testansatz wurde verwendet: 0,7 M Ammoniumchlorid/Ammoniakpuffer (pH 8,5), 0,1 mM NADH, 10 mM Phenylpyruvat und limitierende Mengen an Enzym (10 bis 20 µg Protein pro Test). Die Abnahme der Extinktion von NADH bei 340 nm wurde gemessen. Von den erhaltenen Werten wurde ein Nullwert abgezogen, den man erhielt, wenn der Test ohne Zugabe von Phenylpyruvat ablief. Die Enzymaktivität wird in internationalen Einheiten angegeben, wobei eine Einheit die Abnahme von 1 µmol NADH/min bedeutet.

Brevibacterium species DSM 2448 wächst in kurzen, grampositiven Stäbchen, die mit zunehmendem Alter in kokkoide Formen übergehen. Die Zellen sind unbeweglich und bilden keine Sporen. Wachstum erfolgt strikt aerob. Aus Glucose wird keine Säure gebildet. Katalase- und Nitratreduktion sind positiv, Harnstoffspaltung positiv, Gelantine-, Casein- und Stärkeabbau negativ, $H_2S$-Bildung negativ, Wachstum bei 41 °C negativ. Die Zellwände enthalten meso-Diaminopimelinsäure. Als Zellwandzucker werden Arabinose, Mannose und Galaktose gefunden.

Beispiel 2
Produktion der L-Phenylalanin-Dehydrogenase

Zur Produktion des Enzyms wurde Brevibacterium species DSM 2448 in folgendem Medium angezogen: Glukose 5 g, Hefeextrakt 5 g, Malzextrakt 10 g, L-Phenylalanin 10 g, $KH_2PO_4$ 3 g. Die festen Substrate wurden mit deionisiertem Wasser zu einem Liter gelöst, der pH-Wert dieser Lösung wurde auf 7,2 eingestellt und es wurde 15 Minuten bei 121 °C und 1 bar Überdruck sterilisiert. Die Anzucht erfolgte in 500 ml-Erlenmeyer-Kolben mit zwei Schikanen, gefüllt mit 100 ml Medium. Sterilisiertes Medium wurde beimpft, indem eine Öse voll Brevibacterium species DSM 2448 von einem Schrägagarröhrchen übertragen wurde. Das Medium wurde 20 bis 23 Stunden auf der Rundschüttelmaschine mit 120 Upm bei 27 °C bebrütet. Die gewachsene Kultur wurde zentrifugiert (15 Minuten bei 8 000 × g), das Sediment mit 0,05 M Kaliumphosphatpuffer (pH 7,4) suspendiert. Es wurde erneut zentrifugiert, das so erhaltene gewaschene Sediment in 0,05 M Kaliumphosphatpuffer (pH 7,4) suspendiert, so daß pro Gramm Zellfeuchtmasse 4 ml Puffer verwendet wurden. Diese Suspension wurde am Ultraschallgerät (MSE, 150 Watt, MK2) mit der 3 mm-Spitze unter Kühlung im Eisbad 5 × 1 Minute beschallt mit Pausen zum Abkühlen der Suspension von mindestens 1 Minute. Nach Zentrifugation (10 Minuten bei 12 000 × g) erhielt man einen Rohextrakt, der üblicherweise 1 bis 2 mg Protein/ml und 3 bis 6 Einheiten L-Phenylalanin-Dehydrogenase/ml enthielt. Er kann bei −18 °C aufbewahrt werden, wobei nach einem Monat kein Aktivitätsverlust nachweisbar ist. Falls gewünscht, können niedermolekulare Bestandteile aus dem Rohextrakt durch Diafiltration oder durch Gelchromatographie über Sephadex G-25-Säulen nach üblichen Arbeitsweisen entfernt werden.

Der Verlauf der Enzymbildung während des Wachstums wurde im 1,5 l-Fermenter (Biolafitte) verfolgt. Der Stamm wurde im Produktionsmedium angezogen, zum Animpfen wurden 30 ml einer 24 Stunden alten Vorkultur verwendet.

Wachstumsbedingungen: 30 °C, Belüftungsrate 60 l/h, Turbinenrührer mit 400 Upm.

In Proben wurden gemessen:

a) die optische Dichte (Trübung) der Kultur bei 578 nm als Maß für das Wachstum,

b) der L-Phenylalaningehalt im Medium (nach Abzentrifugieren der Zellen) am Aminosäureanalysator,

c) die abzentrifugierten Zellen wurden aufgeschlossen und in der Suspension (Rohextrakt) die Aktivität der PheDH (U/ml) sowie der Proteingehalt (mg/ml) gemessen und daraus die spezifische Enzymaktivität (U/mg) und die Volumenaktivität der PheDH pro 1 l Kultur berechnet.

Die Daten sind in Abbildung 1 zusammengefasst. Das Enzym wird in einer frühen Wachstumsphase gebildet, mit dem Auftreten der Aktivität sinkt der L-Phe-Gehalt im Medium. Eine maximale Aktivität erreicht man unter diesen Wachstumsbedingungen nach 30 bis 35 Stunden, im weiteren Verlauf sinkt der Enzymgehalt dann auf ca. 50% (65 Stunden).

Beispiel 3
Produktion im 70 l-Maßstab

68 l Produktionsmedium nach Beispiel 2 wurden in einem Fermenter (100 l Volumen, Fabrikat Giovanola Freres SA Monthey) bei 120 °C sterilisiert und nach dem Abkühlen mit 2 l Vorkultur (24 Stunden gewachsen) angeimpft. Die Kultivierung erfolgte bei 30 °C, mit einer Belüftungsrate von 1 vvm und einer Drehzahl von 200 Upm des Turbinenrührers. Nach 24 Stunden Wachstum wurden die Zellen durch Zentrifugation geerntet. Insgesamt wurden 5,9 kg Bakterienfeuchtmasse mit einem Gesamtenzymgehalt von 77 000 Einheiten erhalten. Ein Aliquot dieser Zellmasse wurde aufgeschlossen nach Beispiel 2 und die Aktivität der L-Phenylalanin-Dehydrogenase bestimmt. Die aufgeschlossene Probe zeigte im Rohextrakt eine spezifische Aktivität von 1,6 U/min.

Beispiel 4
Induktion der L-Phenylalanin-Dehydrogenase

Brevibacterium species DSM 2448 wurde in einem Medium mit 1% Malzextrakt, 0,5% Glukose, 2 µg Thiamin/l, 0,3% $KH_2PO_4$ und verschiedenen Aminosäuren, jeweils 1% Konzentration, angezogen. Außerdem wurde L-Phenylalanin-methylester (Konzentration im Medium 0,5%) als Induktor getestet. Der pH-Wert des Mediums betrug 7,4 vor Beginn der Kultivierung. Nach 24 Stunden Wachstum bei 30 °C auf der Rundschüttelmaschine (120 Upm) wurden die Zellen abzentrifugiert, mit Ultraschall aufgeschlossen und mit jedem Extrakt im optischen Test die reduktive Aminierung von 5 α-Ketocarbonsäuren getestet.

Außerdem wurde die Bildung von L-Tryptophan aus Indolylpyruvat am Aminosäureanalysator verfolgt. Indolylpyruvat in der verfügbaren Qualität (Firma Sigma) wies eine zu hohe Adsorption bei 340 nm auf, so daß der optische Test nicht verwendet werden konnte.

Die Ergebnisse sind in der obigen Tabelle 1 zusammengefasst. Darin wird gezeigt, daß im Brevibacterium species DSM 2448 zwei induzierbare Aminosäure-Dehydrogenasen vorliegen. Die L-Phenylalanin-Dehydrogenase setzt Phenylpyruvat, p-Hydroxyphenylpyruvat, Indolylpyruvat und 2-Keto-4-(methylmercapto)butyrat zu den entsprechenden L-Aminosäuren Phenylalanin, Tyrosin, Tryptophan und Methionin um. Diese L-Phenylalanin-Dehydrogenase wird durch L-Phenylalanin, D-Phenylalanin, das Racemat D,L-Phenylalanin, L-Phenylalaninmethylester und L-Histidin induziert. Es fällt auf, daß D-Phenylalanin ein sehr guter Induktor ist. Auffallend ist ferner, daß L-Histidin als Induktor wirkt, obwohl die L-Phenylalanin-Dehydrogenase Imidazolylbrenztraubensäure praktisch nicht als Substrat umsetzt.

Beispiel 5
Aufreinigung der L-Phenylalanin-Dehydrogenase

20 ml Rohextrakt, den man durch Ultraschallaufschluß von 4 g Brevibacterium species DSM 2448 und nachfolgende Zentrifugation erhielt, wurde unter Rühren mit Ammoniumsulfat bis zu 40% Sättigung versetzt und bei 4 °C eine Stunde lang gerührt. Ausgefallenes Protein wurde bei 15 000 × g (30 Minuten) abzentrifugiert. Der Überstand (17,5 ml) wurde auf eine Sephacryl-S 300-Säule (2,5 × 85 cm) aufgetragen, die mit 40%iger Ammoniumsulfatlösung äquilibriert war. Durch Waschen mit 400 ml 0,1 M Kaliumphosphatpuffer (pH 7,5) mit 40% Sättigung Ammoniumsulfat wurde die Hauptmenge an Protein von dieser Säule eluiert. Unter diesen Bedingungen bleiben die Aminosäure-Dehydrogenasen an der Sephacrylsäule gebunden. Wird die Ammoniumsulfatkonzentration durch Anlegen eines Gradienten von 40% auf 10% Sättigung erniedrigt, werden auch die Dehydrogenasen eluiert. Es zeigt sich, daß zuerst bei ca. 32% Ammoniumsulfat-Sättigung die L-Leucin-Dehydrogenase eluiert wird. Deutlich davon abgesetzt bei ca. 26% Ammoniumsulfat-Sättigung wird die L-Phenylalanin-Dehydrogenase eluiert. Das Eluat wird in Fraktionen von 3,6 ml aufgefangen. Die L-Phenylalanin-Dehydrogenase enthaltenden Fraktionen werden kombiniert und durch Ultrafiltration über eine YM5-Membran konzentriert. Die Reinigung ist in Tabelle 2 zusammengefaßt. Das Enzym kann in Gegenwart von 20% Ammoniumsulfat für längere Zeit bei 4 °C stabil gelagert werden.

Beispiel 6
Aufreinigung der L-Phenylalanin-Dehydrogenase

Alternativ zu Beispiel 5 kann Brevibacterium Species DSM 2448 im technischen Maßstab in einer Glasperlmühle aufgeschlossen werden. Als

Tabelle 2

| Reinigungsschritt | Volumen ml | Protein mg | Totalaktivität U | Spez. Aktivität U/mg | Ausbeute % | Anreicherung -fach |
|---|---|---|---|---|---|---|
| Rohextrakt | 20 | 130 | 85 | 0,65 | | |
| Überstand 40%-AS-Fällung | 16,5 | 101 | 81 | 0,80 | 95 | 1,2 |
| Peak II Sephacryl-Säule | 108 | 1,62 | 55 | 34 | 65 | 52 |

Beispiel wurden 270 g Zellen in einer Dyno-Mill Typ KDL aufgeschlossen und die Zelltrümmer mit Hilfe eines wäßrigen 2-Phasen-Systems abgetrennt (gemäß DE-PS 2 639 129). Das verwendete System hatte die Zusammensetzung 20% w/v Zellen, 18% w/w Polyethylenglykol 1540 und 7% w/w Kaliumphosphat, pH 8,0. Die L-Phenylalanin-Dehydrogenase konnte vollständig in die polyethylenglykolreiche Oberphase (1073 ml) extrahiert werden.

Die enzymhaltige Oberphase wurde mit 50 mM Kaliumphosphat-Puffer (pH 7,5), welcher zu 30% gesättigt an Ammoniumsulfat war, verdünnt und mit einer Amicon Hohlfaserpatrone (HIP50) diafiltriert, wobei Fremdproteine und Polyethylenglykol 1540 entfernt wurden. Das Diafiltrat (250 ml) wurde auf eine Sepharose 4B-Säule (5 × 20 cm) aufgetragen, die mit 50 mM Kaliumphosphat-Puffer (pH 7,5), welcher zu 40% gesättigt an Ammoniumsulfat war, äquilibriert wurde. Durch Waschen mit diesem Puffer (1 l) wurde die Hauptmenge an Protein von dieser Säule eluiert, während unter diesen Bedingungen die L-Phenylalanin-Dehydrogenase auf der Säule gebunden blieb. Das Enzym wurde durch Anlegen eines Gradienten entsprechend Beispiel 5 eluiert und anschliessend konzentriert. Die Reinigung nach dieser Methode ist in Tabelle 3 zusammengefaßt.

Tabelle 3

| Reinigungsschritt | Volumen ml | Protein mg | Totalaktivität U | Spez. Aktivität U/mg | Ausbeute % | Anreicherung -fach |
|---|---|---|---|---|---|---|
| Rohextrakt | 650 | 7735 | 6292 | 0,81 | 100 | 1 |
| Top-Phase I | 1073 | 5258 | 6373 | 1,21 | 101 | 1,5 |
| Diafiltration | 250 | 3226 | 3632 | 1,13 | 58 | 1,4 |
| Sepharose 4 B | 210 | 206 | 2680 | 13,0 | 43 | 16,0 |

Beispiel 7

Abhängigkeit der Reaktionsgeschwindigkeit vom pH

Die Reaktionsgeschwindigkeit der reduktiven Aminierung von Phenylpyruvat zu L-Phenylalanin in Gegenwart der L-Phenylalanin-Dehydrogenase wurde in Abhängigkeit vom pH-Wert der Reaktionslösung untersucht. Der Testansatz hatte folgende Zusammensetzung:

0,1 mM NADH, 10 mM Phenylpyruvat und limitierende Mengen an Enzym (Rohextrakt nach Beispiel 2, 20 µg Protein pro Test) in einer 0,7 M Ammoniumchloridlösung bei unterschiedlichen pH-Werten. Die gewählten pH-Werte zwischen 6,75 und 9,0 wurden durch Zugabe von Ammoniak bzw. Salzsäure zu der 0,7 M Ammoniumchloridlösung vor Zusammenmischen des Testansatzes eingestellt.

In Abbildung 2 ist die Reaktionsgeschwindigkeit als Funktion des pH-Wertes aufgetragen. Das pH-Optimum liegt bei 8,5.

Die Reaktionsgeschwindigkeit der oxidativen Desaminierung von L-Phenylalanin, katalysiert durch die L-Phenylalanin-Dehydrogenase, wurde gleichfalls in Abhängigkeit vom pH-Wert untersucht. Der Testansatz hatte folgende Zusammensetzung:

4,0 mM L-Phenylalanin und 3 mM $NAD^+$ in einem 0,1 M Glycin/NaCl-Puffer bei unterschiedlichen pH-Werten. Es wurden mehrere pH-Werte im Bereich 6,0 bis 10,5 durch Zugabe von Salzsäure oder Natronlauge zu dem Glycin/NaCl-Puffer vor Zusammenmischen des Testansatzes eingestellt.

Die Ergebnisse sind zusammenfassend gleichfalls in Abbildung 2 dargestellt. Die Rückreaktion steigt bis pH 10,5 an.

Beispiel 8

Abhängigkeit der Reaktionsgeschwindigkeit von den Substratkonzentrationen

Die Abhängigkeit der Reaktionsgeschwindigkeit der reduktiven Aminierung von Phenylpyruvat zu L-Phenylalanin für das Substrat NADH wurde in folgendem Testansatz untersucht:

0,7 M Ammoniumchlorid/Ammoniak-Puffer (pH 8,5), 10 mM Phenylpyruvat, limitierende Mengen an Enzym (Rohextrakt nach Beispiel 2, 20 µg Protein pro Test). Die NADH-Konzentration im Testansatz wurde im Bereich von 0,025 bis 0,35 mM variiert.

Es zeigte sich, daß die optimale Reaktionsgeschwindigkeit bei 0,3 mM erreicht wird. Der $K_M$-Wert für NADH beträgt 0,064 mM.

Die Abhängigkeit der Reaktionsgeschwindigkeit der reduktiven Aminierung von Phenylpyruvat zu L-Phenylalanin von der Ammoniumionen-Konzentration wurde in folgendem Testansatz untersucht:

10 mM Phenylpyruvat, 0,2 mM NADH, limitierende Mengen an Enzym (Rohextrakt nach Beispiel 2, 20 µg Protein pro Test). Es wurde ein Ammonium-Chlorid-Puffer eingesetzt, dessen pH-Wert mit Ammoniak auf pH 8,5 eingestellt war. Die Ammoniumchloridmolarität im Test wurde im Bereich von 0,095 bis 0,7 M variiert.

Es zeigte sich, daß die Reaktionsgeschwindigkeit bis mindestens 0,5 M Ammoniumchlorid ansteigt. 0,7 M Ammoniumchlorid wirken nicht inhibierend und werden daher für den Test des Enzyms als optimal angesehen.

Die reduktive Aminierung verschiedener α-Ketocarbonsäuren wurde in Abhängigkeit von der Ketosäurekonzentration untersucht. Dazu wurde folgender Textansatz verwendet:

0,7 M Ammoniumchlorid/Ammoniak-Puffer (pH 8,5), 0,2 mM NADH, limitierende Mengen an Enzym (1 µg nach Beispiel 5 gereinigtes Protein). Die Ketosäurekonzentration wurde jeweils im Bereich von 0,01 bis 30 mM variiert.

Die Anfangsreaktionsgeschwindigkeit (Δ Extinktion 340 nm/Minute) wird nach Michaelis-Menten ausgewertet. Die gefundenen $K_M$- und $V_{max}$-Werte sind in Tabelle 4 zusammengefaßt. Wegen der Störung des optischen Testes im Falle des Substrates Indolylpyruvat wurde bei der reduktiven Aminierung von Indolylpyruvat zu L-Tryptophan die gebildete L-Tryptophanmenge als Funktion der Zeit am Aminosäureanalysator bestimmt (Biotronik BC 6000, ausgerüstet mit einem Integrator Biotronik, System 1, in einem 1-Säulen-Programm, als Eichlösung wurde ein Aminosäure-Standard IV der Firma Pierce verwendet).

Tabelle 4

$K_M$- und $V_{max}$-Werte mit gereinigter PheDH (0,055 mg Protein/ml)

| Substrat | $K_M$ [mM] | $V_{max}$ [U/ml] |
|---|---|---|
| Phenylpyruvat | 0,11 | 1,82 |
| p-Hydroxyphenylpyruvat | 0,24 | 1,75 |
| 2-Keto-4-(methylmercapto)-butyrat | 3,0 | 1,08 |
| Indolylpyruvat | 8,0 | 0,44 |

Die Abhängigkeit der Reaktionsgeschwindigkeit der oxidativen Desaminierung von L-Phenylalanin von der $NAD^+$-Konzentration wurde in folgendem Testansatz untersucht:

0,1 M Glycin-NaCl/NaOH-Puffer (pH 10,7), 4 mM L-Phenylalanin, limitierende Mengen an Enzym (20 µg nach Beispiel 6 gereinigtes Protein).

Die $NAD^+$-Konzentration wurde im Bereich von 0,1 bis 5,0 mM variiert. Es zeigte sich, daß der optimale Umsatz bei einer Konzentration von 3 mM erreicht wird.

Die Abhängigkeit der Reaktionsgeschwindigkeit der oxidativen Desaminierung von der L-Phenylalanin-Konzentration wurde in folgendem Testansatz untersucht:

0,1 M Glycin-NaCl/NaOH-Puffer (pH 10,7) 3 mM $NAD^+$, limitierende Mengen an Enzym (20 µg nach Beispiel 6 gereinigtes Protein). Die Phenylalanin-Konzentration wurde im Bereich von 0,3 bis 15 mM variiert.

Das bei der Reaktion entstandene NADH wurde bei 340 nm gemessen. Die Anfangs-Reaktionsgeschwindigkeit wurde nach Michaelis-Menten ausgewertet. Der $K_M$-Wert für Phenylalanin beträgt 0,8 mM, die maximale Reaktionsgeschwindigkeit beträgt 1,02 U/min.

Beispiel 9

Herstellung von L-Phenylalanin, L-Tyrosin, L-Tryptophan und L-Methionin durch reduktive Aminierung der analogen α-Ketocarbonsäuren

Um das bei der reduktiven Aminierung verbrauchte NADH zu regenerieren, wird dem Reaktionsansatz ein Überschuß an Ammoniumformiat zugesetzt und das Enzym Formiat-Dehydrogenase (E.C. 1.2.1.2), das bei der Oxidation von Formiat zu $CO_2$ das Cosubstrat $NAD^+$ zu NADH reduziert.

Die reduktive Aminierung einer Reihe von α-Ketocarbonsäuren wurde unter vergleichenden Bedingungen getestet. Die Testansätze enthielten dabei einheitlich 400 mM Ammoniumformiat (pH 8,5), 0,3 mM NADH, 0,6 U/ml Formiat-Dehydrogenase (Präparat gemäß Kroner et al. (1982), J. Chem. Tech. Biotechnol. 32, 130 bis 137), 0,5 U/ml L-Phenylalanin-Dehydrogenase, 25 mM α-Ketocarbonsäure. Das Gesamtvolumen betrug 3 ml. Die Inkubation erfolgte unter Rühren bei 27 °C. Die Produktbildung wurde jeweils am Aminosäureanalysator verfolgt. Die Ergebnisse sind in Tabelle 5 zusammengefaßt. Phenylpyruvat, p-Hydroxyphenylpyruvat, Indolylpyruvat und 2-Keto-4-(methylmercapto)-butyrat werden gut umgesetzt. Dagegen wird Imidazolylpyruvat praktisch nicht als Substrat verwertet.

Beispiel 10

Kontinuierliche Herstellung von L-Phenylalanin

Eine kontinuierliche Synthese von Phenylalanin aus Phenylpyruvat ist möglich in einem Enzymmembranreaktor unter Verwendung von molekulargewichtsvergrössertem, an Polyethylenglykol (PEG) gebundenem NADH. Das PEG-NADH wird gemäß DE-PS 2 841 414 hergestellt. Das modifizierte Coenzym und die eingesetzten Enzyme Formiat-Dehydrogenase und L-Phenylalanin-Dehydrogenase werden durch eine Ultrafiltrationsmembran YM5 (Produkt der Firma Amicon, Witten) im Reaktor zurückgehalten, während die niedermolekularen Bestandteile der Reaktionslösung, nicht umgesetztes Substrat und das gebildete L-Phenylalanin, laufend aus der Lösung entfernt werden. Das Reaktorvolumen wird konstant ge-

Tabelle 5

| Substrat | Produkt | Produktbildung nach 2 Stunden mM % | 6 Stunden mM % |
|---|---|---|---|
| Phenylbrenztraubensäure | L-Phenylalanin | 12,5 (50) | 16,6 (66) |
| p-Hydroxyphenylbrenztraubensäure | L-Tyrosin | 10,2 (41) | 14,8 (59) |
| Indolylbrenztraubensäure | L-Tryptophan | 6,2 (25) | 12,0 (48) |
| Imidazolylbrenztraubensäure | L-Histidin | 0,2 (1) | 0,5 (2) |
| 2-Keto-4-(methylmercapto)-buttersäure | L-Methionin | 12,8 (51) | 15,6 (62) |

halten, indem im gleichen Maß aus einem Reservoir Phenylpyruvat und Ammoniumformiat nachdosiert wird, in dem das Ultrafiltrat den Reaktor verläßt. Das Reaktorvolumen betrug 10 ml, die Konzentration an PEG-NADH 0,3 mM, es wurden 20 Einheiten Formiat-Dehydrogenase und 20 Einheiten L-Phenylalanin-Dehydrogenase in den Reaktor eingespült. Die Substratlösung enthielt 400 mM Ammoniumformiat pH 8,3 und 20 mM Phenylpyruvat (Natriumsalz).

Die Reaktionslösung wurde mit einer Schlauchpumpe laufend mit 30 ml/Stunde über die Membran gepumpt. Etwa 4 ml Ultrafiltrat wurden pro Stunde erhalten. Das entspricht einer Verweilzeit von 2,5 Stunden. Mit diesem Versuchsaufbau konnten im Verlauf von 110 Stunden 9,8 mMol (1,83 g) Natriumphenylpyruvat durchgesetzt werden. Das Ultrafiltrat wurde in Fraktionen aufgefangen und der Gehalt an L-Phenylalanin am Aminosäureanalysator bestimmt. Über einen Zeitraum von 110 Stunden wurden im Mittel 70% des eingesetzten Phenylpyruvates zu L-Phenylalanin umgesetzt, in den vereinigten Ultrafiltraten wurden 6,9 mMol (1,14 g) L-Phenylalanin gemessen. Das in diesem Beispiel erhaltene Reaktionsprodukt ist relativ einfach zu reinigen, da die Lösung nur mit nicht umgesetztem Phenylpyruvat und Ammoniumformiat verunreinigt ist. Das Ultrafiltrat (450 ml, 1,14 g L-Phenylalanin) wurde lyophilisiert, mit 50 ml 0,5 M Ameisensäure aufgenommen und ein Aliquot von 12,5 ml auf eine Ionenaustauschersäule (Biorad AG 50 W X8, 200 bis 400 mesh, $H^+$-Form, 1 × 10 cm) aufgetragen. Die Säule wurde mit 100 ml 0,5 M Ameisensäure gewaschen. Unter diesen Bedingungen wird Phenylpyruvat nicht gebunden. Durch eine Farbreaktion mit Eisen III-Chlorid-Lösung (7,5%, pH 2,5, Messung der Adsorption bei 436 nm) konnte gezeigt werden, daß Phenylpyruvat nach Passieren von 20 bis 25 ml Waschlösung von der Säule entfernt war. L-Phenylalanin wird von dem Ionenaustauscher mit einer 5%igen Pyridinlösung eluiert. Unter diesen Umständen bleiben Ammoniumionen am Ionenaustauscher gebunden, so daß der L-Phenylalanin-Nachweis im Eluat mit Ninhydrin möglich ist. Der Austauscher wird durch Behandlung mit 4N Salzsäure regeneriert und wird nach Waschen mit deionisiertem Wasser wieder eingesetzt. Die Phenylalanin enthaltenden Eluate wurden vereinigt und am Rotationsverdampfer im Vakuum zur Trockne eingeengt. Zur vollständigen Entfernung des Pyridins wurde mehrmals mit Wasser aufgenommen. Auf diese Weise wurden 1,12 g L-Phenylalanin isoliert, die für weitere analytische Bestimmungen eingesetzt wurden.

Aminosäureanalyse: Der Aminosäureanalysator zeigte, daß das Produkt aus Beispiel 10 außer L-Phenylalanin keine weiteren Aminosäuren enthielt.

Optische Drehung: Für die Messungen wurde ein Perkin-Elmer Polarimeter Typ 241 eingesetzt, die Messungen wurden bei 436 nm, pH 2,1 und 30 °C durchgeführt. Eine Eichkurve mit L-Phenylalanin zeigt einen linearen Zusammenhang von Drehwert und Konzentration zwischen 5 und 100 mM. Der Drehwert der in Beispiel 10 synthetisierten Probe betrug −0,371° in einer 71 mM Lösung (Konzentrationsbestimmung über den Aminosäureanalysator). Ein vergleich mit der Eichkurve zeigt, daß optisch reines L-Phenylalanin erhalten wurde.

Test auf D-Aminosäure mittels D-Aminosäureoxidase (d-AOD): D-AOD aus Schweinenieren (Produkt Boehringer Mannheim GmbH) ist spezifisch für D-Aminosäuren. Das bei der Oxidation von D-Aminosäuren mit D-AOD entstehende Wasserstoffperoxid wird mit Hilfe des Enzyms Peroxidase auf einen Leukofarbstoff übertragen. Es wird eine Eichreihe mit D-Phenylalanin aufgenommen, die im Bereich 0,01 bis 0,35 mM einen linearen Zusammenhang zwischen der Differenz der Adsorptionswerte bei 436 nm und der Konzentration an D-Phenylalanin zeigt. Die Nachweisgrenze liegt bei 0,005 mM. Die eingesetzte Probe (55 mM L-Phenylalanin nach Beispiel 10) war in diesem Test inaktiv. Verunreinigungen mit D-Phenylalanin müssen daher unter 0,01% liegen.

**Patentansprüche**

1. Mikrobiologisch hergestellte L-Phenylalanin-Dehydrogenase, gekennzeichnet durch die folgenden Eigenschaften:

a) katalysiert die reduktive Aminierung von Phenylbrenztraubensäure zu L-Phenylalanin in Gegenwart von Ammoniumionen und mit NADH (Nicotinamid-adenin-dinucleotid) als Coenzym,

b) katalysiert die reduktive Aminierung weiterer α-Ketocarbonsäuren zu den entsprechenden α-Aminocarbonsäuren, insbesondere von p-Hydroxyphenylbrenztraubensäure zu L-Tyrosin, von Indolylbrenztraubensäure zu L-Tryptophan und von 2-Keto-4-(methylmercapto)-buttersäure zu L-Methionin, in Gegenwart von Ammoniumionen und mit NADH als Coenzym,

c) katalysiert die oxidative Desaminierung von L-Phenylalanin, L-Tyrosin, L-Tryptophan und L-Methionin mit $NAD^+$ als Coenzym,

d) einen optimalen pH-Bereich für die reduktive Aminierung von 8,5 ± 1 und

e) einen optimalen pH-Bereich für die oxidative Desaminierung von 10 ± 1

und erhältlich durch Züchtung des Stammes Brevibacterium DSM 2448 und Gewinnung daraus.

2. Verfahren zur Gewinnung der L-Phenylalanin-Dehydrogenase gemäß Anspruch 1, dadurch gekennzeichnet, daß man Brevibacterium species DSM 2448 in einem wäßrigen Nährmedium, das eine Quelle für Kohlenstoff und Stickstoff, Thiamin, Mineralsalze, sowie einen Induktor enthält, bei einem pH zwischen 6,5 und 7,5 und einer Temperatur zwischen 25 und 32 °C aerob kultiviert, die Zellmasse abtrennt und das Enzym aus den Zellen isoliert.

3. Verfahren nach Anspruch 2, dadurch gekennzeichnet, daß man L-Phenylalanin, D-Phenylalanin, D,L-Phenylalanin, einen D,L-Phenylalaninester oder L-Histidin als Induktor einsetzt.

4. Verfahren nach Anspruch 2 oder 3, dadurch gekennzeichnet, daß man die Zellen mechanisch

aufschließt, die unlöslichen Zelltrümmer abtrennt und das Enzym in an sich bekannter Weise weiter anreichert.

5. Verfahren nach Anspruch 4, dadurch gekennzeichnet, daß man die Anreicherung des Enzyms durch fraktionierte Salzfällung vornimmt.

6. Verfahren nach Anspruch 4, dadurch gekennzeichnet, daß man die Anreicherung des Enzyms durch ein chromatographisches Trennverfahren vornimmt.

7. Verwendung der L-Phenylalanin-Dehydrogenase gemäß Anspruch 1 zur enzymatischen Umwandlung von Phenylbrenztraubensäure, p-Hydroxyphenylbrenztraubensäure, Indolylbrenztraubensäure oder 2-Keto-4-(methylmercapto)-buttersäure in die entsprechenden L-α-Aminocarbonsäuren.

## Claims

1. Microbiologically prepared L-phenylalanine dehydrogenase, characterized by the following properties:

a) catalyses the reductive amination of phenylpyruvic acid to L-phenylalanine in the presence of ammonium ions and with NADH (nicotinamide adenine dinucleotide) as coenzyme,

b) catalyses the reductive amination of other α-keto carboxylic acids to the corresponding α-amino carboxylic acids, especially of p-hydroxyphenylpyruvic acid to L-tyrosine, of indolylpyruvic acid to L-tryptophan, and of 2-keto-4-(methylmercapto)-butyric acid to L-methionine, in the presence of ammonium ions and with NADH as coenzyme,

c) catalyses the oxidative deamination of L-phenylalanine, L-tyrosine, L-tryptophan and L-methionine with $NAD^+$ as coenzyme,

d) an optimal pH range for the reductive amination of 8.5 ± 1, and

e) an optimal pH range for the oxidative deamination of 10 ± 1

and obtainable by cultivating the strain Brevibacterium DSM 2448 and obtaining therefrom.

2. Process for obtaining the L-phenylalanine dehydrogenase according to Claim 1, characterized in that Brevibacterium species DSM 2448 is cultivated aerobically in an aqueous nutrient medium which contains a source for carbon and nitrogen, thiamine, mineral salts and an inducer, at a pH between 6.5 and 7.5 and a temperature between 25 and 32 °C, the biomass is separated off, and the enzyme is isolated from the cells.

3. Process according to Claim 2, characterized in that L-phenylalanine, D-phenylalanine, D,L-phenylalanine, a D,L-phenylalanine ester or L-histidine is used as inducer.

4. Process according to Claim 2 or 3, characterized in that cells are mechanically disrupted, while the insoluble cell debris is removed, and the enzyme is further enriched in a manner known per se.

5. Process according to Claim 4, characterized in that the enrichment of the enzyme is carried out by fractional salt precipitation.

6. Process according to Claim 4, characterized in that the enrichment of the enzyme is carried out by a chromatographic separation process.

7. Use of the L-phenylalanine dehydrogenase according to Claim 1 for the enzymatic conversion of phenylpyruvic acid, p-hydroxyphenylpyruvic acid, indolylpyruvic acid or 2-keto-4-(methylmercapto)-butyric acid into the corresponding L-α-amino carboxylic acids.

## Revendications

1. L-phénylalanine-déshydrogénase produite par voie microbiologique, caractérisée par les propriétés suivantes:

a) elle catalyse l'amination réductrice de l'acide phénylpyruvique en L-phénylalanine, en présence d'ions ammonium et avec du NADH (nicotinamide-adénine-dinucléotide) en tant que coenzyme;

b) elle catalyse l'amination réductrice d'autres acides α-cétocarboxyliques en les acides α-aminocarboxyliques correspondants, en particulier de l'acide p-hydroxyphénylpyruvique en L-tyrosine, de l'acide indolylpyruvique en L-tryptophane et de l'acide 2-céto-4-(méthylmercapto)-butyrique en L-méthionine, en présence d'ions ammonium et avec du NADH en tant que coenzyme;

c) elle catalyse la désamination oxydante de la L-phénylalanine, de la L-tyrosine, du L-tryptophane et de la méthionine avec du $NAD^+$ ent tant que coenzyme;

d) elle a une zone optimale de pH pour l'amination réductrice égale à 8,5 ± 1;

e) elle a une zone optimale de pH pour la désamination oxydante égale à 10 ± 1;

et pouvant être obtenue par culture de la souche Brevibacterium DSM 2448 et extraction à partir de celle-ci.

2. Procédé pour l'obtention de la L-phénylalanine-déshydrogénase selon la revendication 1, caractérisé en ce que l'on soumet à une culture aérobie, à un pH entre 6,5 et 7,5 et à une température comprise entre 25 et 32 °C, Brevibacterium sp. DSM 2448 dans un milieu nutritif aqueux qui contient une source de carbone et d'azote, de la thiamine, des sels minéraux, ainsi qu'un inducteur, on sépare la masse cellulaire et on isole l'enzyme à partir des cellules.

3. Procédé selon la revendication 2, caractérisé en ce que l'on utilise en tant qu'inducteur la L-phénylalanine, la D-phénylalanine, la D,L-phénylalanine, un ester de la D,L-phénylalanine, ou la L-histidine.

4. Procédé selon la revendication 2 ou 3, caractérisé en ce que l'on rompt mécaniquement les cellules, on sépare les débris cellulaires insolubles et on enrichit davantage l'enzyme d'une façon connue en soi.

5. Procédé selon la revendication 4, caractérisé en ce que l'on effectue l'enrichissement de l'enzyme par précipitation fractionnée au moyen d'un sel.

6. Procédé selon la revendication 4, caractérisé en ce que l'on effectue l'enrichissement de l'enzyme au moyen d'un procédé de séparation chromatographique.

7. Utilisation de la L-phénylalanine-déshydrogénase selon la revendication 1 pour la conversion enzymatique de l'acide phénylpyruvique, de l'acide p-hydroxyphénylpyruvique, de l'acide indolylpyruvique ou de l'acide 2-céto-4-(méthylmercapto)-butyrique en les acides L-α-aminocarboxyliques correspondants.

Abb. 1

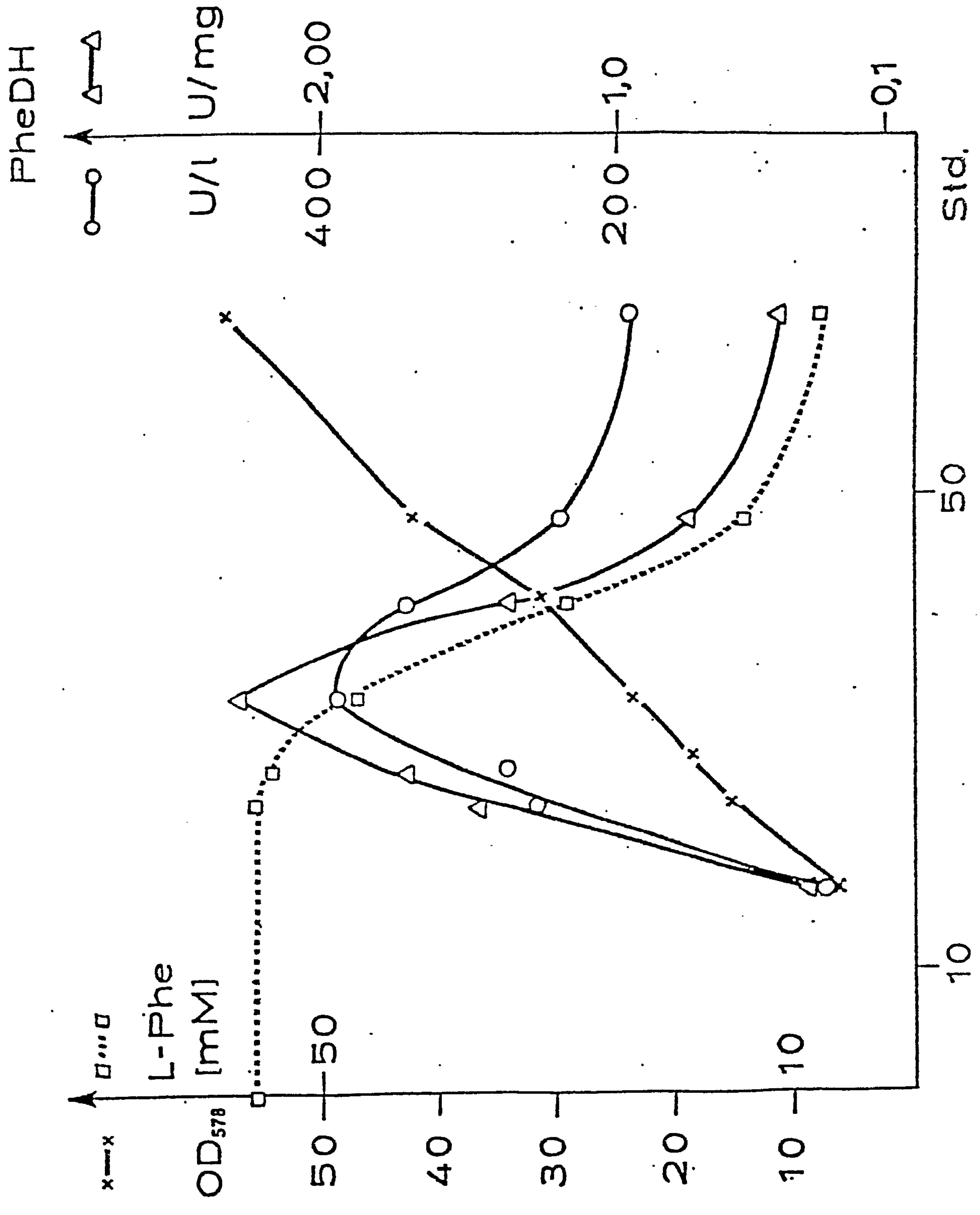
PheDH
U/l
U/mg
400
200
2,00
1,0
0,1
Std.
10
50
L-Phe
[mM]
50
10
$OD_{578}$
50
40
30
20
10

Abb. 2

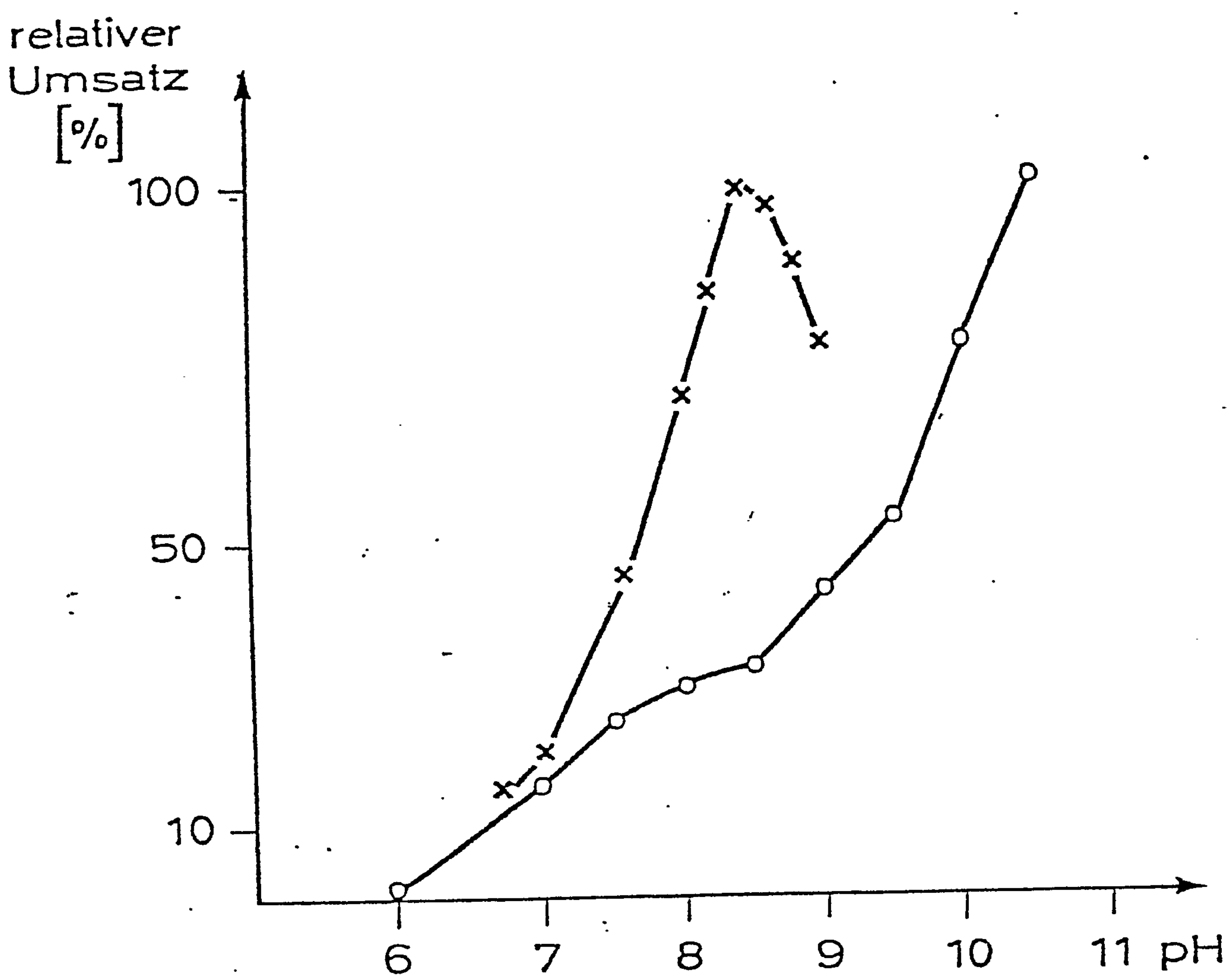


x —— x: reduktive Aminierung
o —— o: oxidative Desaminierung